# EUROPEAN PATENT APPLICATION

(11) **EP 4 101 609 A1**
(43) Date of publication of application: **14.12.2022**
(21) Application number: 20917489.5
(22) Date of filing: 19.11.2020
(51) Int. Cl.: B26B 19/48

(54) **ELECTRIC SHAVER**

(30) Priority: 04.02.2020 JP 2020017021
(71) Applicant: Panasonic Intellectual Property Management Co., Ltd., Osaka-shi, Osaka 540-6207 (JP)
(72) Inventor: KANAMORI, Yoshiaki, Osaka-shi, Osaka (JP); NARITA, Kenji, Osaka-shi, Osaka (JP)
(74) Representative: Grünecker Patent- und Rechtsanwälte PartG mbB
(86) International application number: PCT/JP2020/043174
(87) International publication number: WO 2021/157160

(57) **Abstract**

An electric razor comprises a head, a grip, a blade unit disposed in the head for cutting hair, a skin electrode disposed in the head, and a grip electrode disposed in the grip. The electric razor further comprises a voltage application circuit for applying a voltage between the skin electrode and the grip electrode to cause a current to flow between the skin electrode and the grip electrode through a human body, and a controller that controls the voltage application circuit.

## Description

### TECHNICAL FIELD

The present disclosure relates to an electric razor.

### BACKGROUND ART

An ion introduction device for enhanced transport of substances through a region of a skin and having an electrode system and an associated controller in order to generate two transport electric fields directed in different directions in the region is described in PTL 1.

Such an ion introduction device is also used in an electric razor.

### Citation List

### Patent Literature

PTL 1: Unexamined Japanese Patent Publication No. 2010-510821

### SUMMARY OF THE INVENTION

In the electric razor of the related art, hair is cut and shaved with a blade such as an inner blade and an outer blade. However, when shaving is performed with the electric razor, a rough skin state represented by razor rash may occur. Thus, there is a demand for a countermeasure to more effectively reduce the razor rash and a countermeasure to more efficiently care for a skin surface for which hair is removed.

The present disclosure has been made in view of such problems of the prior art. An object of the present disclosure is to provide an electric razor that can more effectively reduce razor rash and more efficiently care for a skin surface to be shaved.

An electric razor according to a first aspect of the present disclosure comprises a head, a grip, a blade unit that is disposed in the head for cutting hair, a first skin electrode that is disposed at a position of the head touching a skin surface of a human body during cutting of hair, and a grip electrode that is disposed at a position of the grip touching a finger of the human body during the cutting of hair. The electric razor further comprises a voltage application circuit for applying a voltage between the first skin electrode and the grip electrode to cause a current to flow between the first skin electrode and the grip electrode through the human body, and a controller that controls the voltage application circuit.

An electric razor according to a second aspect of the present disclosure comprises a head, a grip, a blade unit that is disposed in the head for cutting hair, a first skin electrode that is disposed at a position of the head touching a skin surface of a human body during cutting of hair, and a grip electrode that is disposed at a position of the grip touching a finger of the human body during cutting of hair. The electric razor further comprises a voltage application circuit for applying a voltage between the first skin electrode and the grip electrode to cause a current to flow between the first skin electrode and the grip electrode through the human body, and a controller that controls the voltage application circuit. A heater is incorporated in the first skin electrode.

According to the present disclosure, it is possible to provide the electric razor that can more effectively reduce the razor rash and more efficiently care for the skin surface to be shaved.

### BRIEF DESCRIPTION OF THE DRAWINGS

Fig. 1 is a perspective view illustrating an electric razor according to an exemplary embodiment.
Fig. 2 is an enlarged perspective view illustrating a main part of the electric razor according to the exemplary embodiment.
Fig. 3 is a block diagram illustrating a functional configuration of the electric razor according to the exemplary embodiment.
Fig. 4 is a waveform chart illustrating an example of a voltage (pulses) to be applied by a voltage application circuit in an ion introduction mode.
Fig. 5 is a waveform chart illustrating an example of a voltage (pulses) to be applied by the voltage application circuit in an electroporation mode.
Fig. 6 is a waveform chart illustrating an example of a voltage (pulses) to be applied by the voltage application circuit in a micro-current mode.
Fig. 7 is a diagram schematically illustrating a skin electrode including a suspension mechanism.
Fig. 8 is a diagram schematically illustrating a skin electrode including a pop-up mechanism.
Fig. 9 is an enlarged perspective view of a main part of an electric razor illustrating a skin electrode according to a modification example.
Fig. 10 is an enlarged perspective view of a main part of an electric razor illustrating a skin electrode according to another modification example.

### DESCRIPTION OF EMBODIMENT

Hereinafter, an exemplary embodiment will be described in detail with reference to the drawings. However, unnecessarily detailed description is omitted in some cases. For example, a detailed description of already well-known matters or a redundant description of substantially the same configuration may be omitted.

Note that, the accompanying drawings and the following description are only presented to help those skilled in the art fully understand the present disclosure, and are not intended to limit the subject matters as claimed in the claims.

Hereinafter, an electric razor for removing hair (shaving) and simultaneously caring for a skin surface will be exemplified.

Hereinafter, a width direction of the electric razor in front view is referred to as left-right direction X, a depth direction of the electric razor in front view is referred to as front-back direction Y, and a height direction of the electric razor in front view is referred to as up-down direction Z.

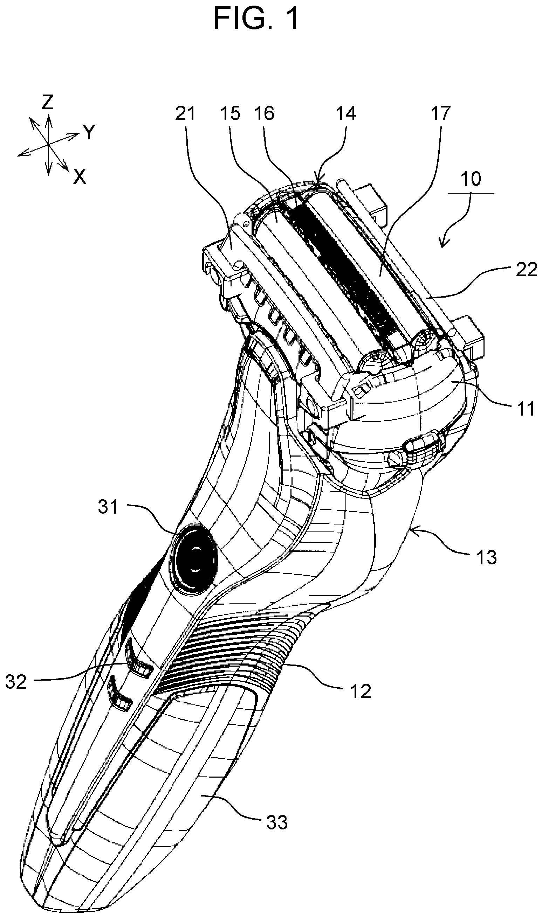

Hereinafter, electric razor 10 according to the present exemplary embodiment will be described with reference to Figs. 1 to 6.

### [1. Configuration]

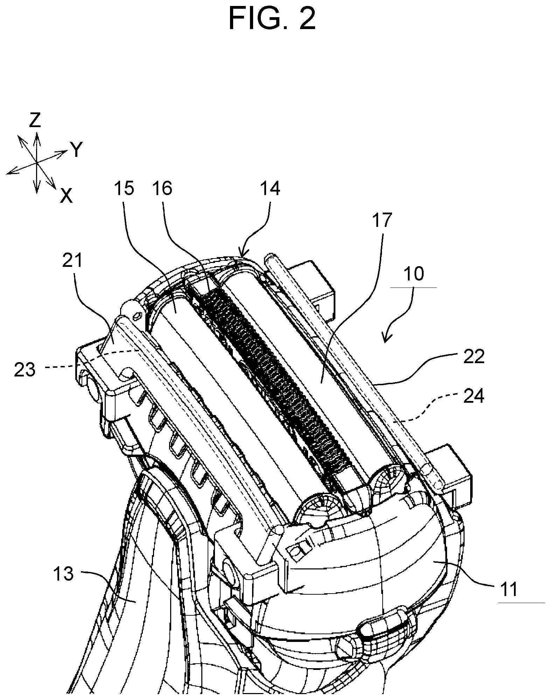

As illustrated in Figs. 1 and 2, electric razor 10 according to the present exemplary embodiment includes head 11 and electric razor body 13 having grip 12.

Blade unit 14 for cutting hair such as beard and hair is disposed and held in head 11. Blade unit 14 includes outer net blade (first outer net blade) 15, outer slit blade 16, and outer net blade (second outer net blade) 17. In the present exemplary embodiment, first outer net blade 15, outer slit blade 16, and second outer net blade 17 are disposed in head 11 in this order from a near side in front-back direction Y.

Skin electrode (first skin electrode) 21 for applying a current to a skin surface of a user and skin electrode (second skin electrode) 22 different from first skin electrode 21 are disposed in head 11. First skin electrode 21 and second skin electrode 22 are disposed at positions of head 11 touching the skin surface of the user during shaving.

First skin electrode 21 and second skin electrode 22 are made of a conductor disposed in head 11. An electrical insulation treatment is performed on a surface of the conductor of second skin electrode 22 in order to perform energization of electroporation (electric punch method), which will be described later.

Heater (first heater) 23 is incorporated in first skin electrode 21, and heater (second heater) 24 different from first heater 23 is incorporated in second skin electrode 22. Driving of first heater 23 and second heater 24 is controlled by controller 36, which will be described later.

In the present exemplary embodiment, first skin electrode 21 and second skin electrode 22 are disposed in head 11 in this order from the near side in front-back direction Y, and first skin electrode 21 and second skin electrode 22 are disposed at an interval in front-back direction Y.

Contrary to the present exemplary embodiment, second skin electrode 22 and first skin electrode 21 may be disposed in head 11 in this order from the near side in front-back direction Y.

Blade unit 14 (first outer net blade 15, outer slit blade 16, and second outer net blade 17) is disposed between first skin electrode 21 and second skin electrode 22. That is, first skin electrode 21 and second skin electrode 22 are disposed at an interval equal to or longer than a dimension in front-back direction Y of blade unit 14 with respect to front-back direction Y.

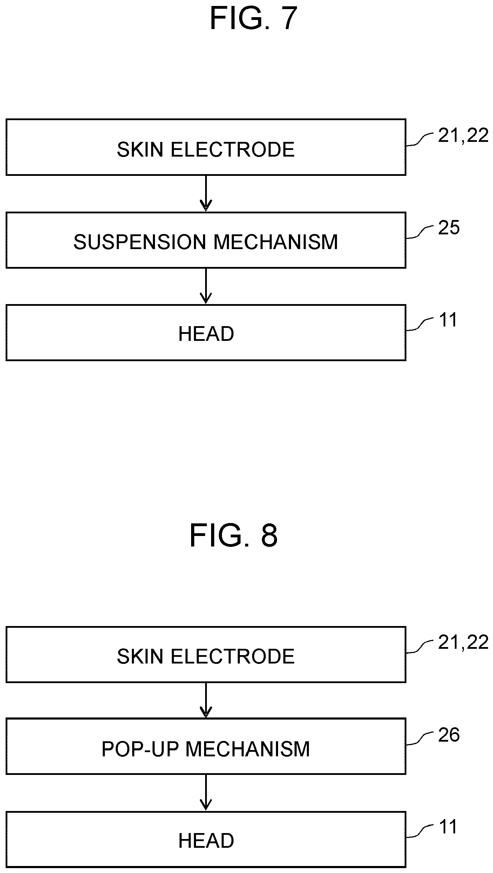

As illustrated in Fig. 7, suspension mechanism 25 may be interposed between the skin electrodes (first skin electrode 21 and second skin electrode 22) and head 11. That is, skin electrodes 21 and 22 may include suspension mechanism 25 interposed between skin electrodes 21 and 22 and head 11. For example, partial touch between skin electrodes 21 and 22 and the skin surface of the user is suppressed by suspension mechanism 25 by interposing suspension mechanism 25 between skin electrodes 21 and 22 and head 11, and skin electrodes 21 and 22 can stably touch the skin surface.

As illustrated in Fig. 8, pop-up mechanism 26 may be interposed between the skin electrodes (first skin electrode 21 and second skin electrode 22) and head 11. That is, skin electrodes 21 and 22 may have pop-up mechanism 26 interposed between skin electrodes 21 and 22 and head 11. As a result, for example, skin electrodes 21 and 22 can be popped up upward in up-down direction Z by pop-up mechanism 26, and blade unit 14 can cause skin electrodes 21 and 22 to touch the skin surface of the user without touching the skin surface.

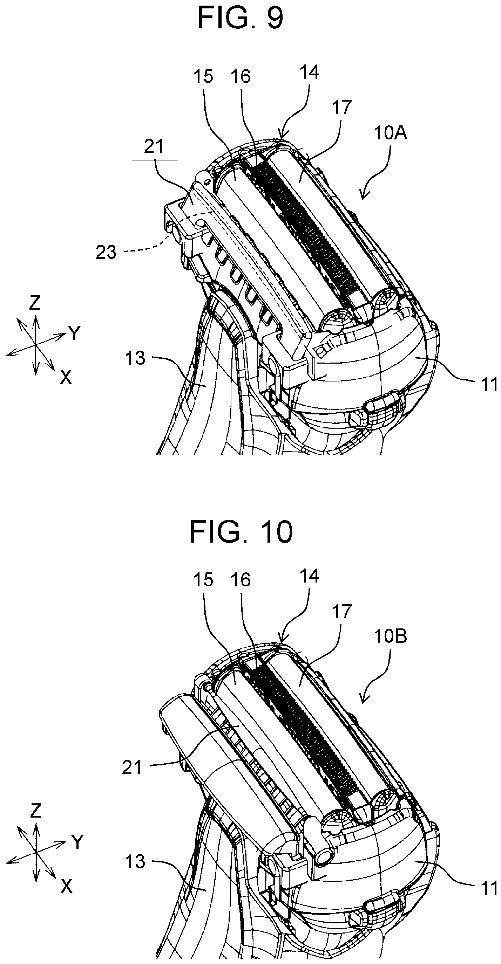

As in electric razor 10A in Fig. 9, only skin electrode (first skin electrode) 21 for applying a current to the skin surface of the user may be disposed in head 11. In Fig. 9, first skin electrode 21 is disposed on the near side in front-back direction Y with respect to blade unit 14. However, for example, first skin electrode 21 may be disposed in blade unit 14, or may be disposed behind blade unit 14 in front-back direction Y. In some cases, a plurality of first skin electrodes 21 may be disposed in head 11 at intervals in left-right direction X.

Furthermore, as in electric razor 10B illustrated in Fig. 10, roller-shaped skin electrode (first skin electrode) 21 may be disposed in head 11. That is, first skin electrode 21 may be formed in a roller shape in order to function as a beauty treatment roller. In Fig. 10, roller-shaped first skin electrode 21 is disposed on the near side in front-back direction Y with respect to blade unit 14. However, for example, first skin electrode 21 may be disposed in blade unit 14, or may be disposed behind blade unit 14 in front-back direction Y.

As illustrated in Fig. 1, power switch 31 for operating electric razor 10, and changeover switch 32 for switching between skin care modes, which will be described later, are provided in electric razor body 13.

Electric razor body 13 doubles as grip 12 of electric razor 10, and grip electrode 33 is disposed in grip 12. Grip electrode 33 is disposed at a position of grip 12 where the grip electrode touches a finger of the user during shaving.

Grip electrode 33 is made of a conductor disposed on a surface of grip 12.

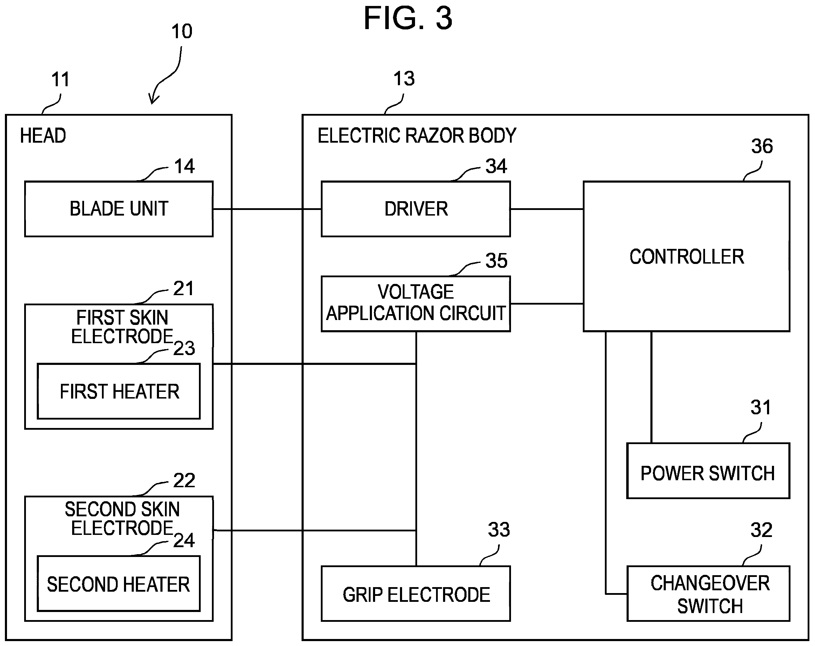

Furthermore, driver (motor) 34, voltage application circuit 35, controller 36, and the like are housed in electric razor body 13 (see Fig. 3).

Next, a functional configuration of electric razor 10 will be described with reference to Fig. 3.

As illustrated in Fig. 3, electric razor 10 includes driver 34 for driving blade unit 14, and voltage application circuit 35 for applying voltages to first skin electrode 21, second skin electrode 22, and grip electrode 33.

Electric razor 10 includes controller 36. Controller 36 receives signals from power switch 31 and changeover switch 32, and controls driver 34, voltage application circuit 35, and the like according to the signals.

### [2. Operation]

Next, operations and effects of electric razor 10 according to the present exemplary embodiment will be described.

Controller 36 has a plurality of modes as the skin care modes. In the present exemplary embodiment, controller 36 has three skin care modes of an ion introduction mode, an electroporation mode, and a microcurrent mode.

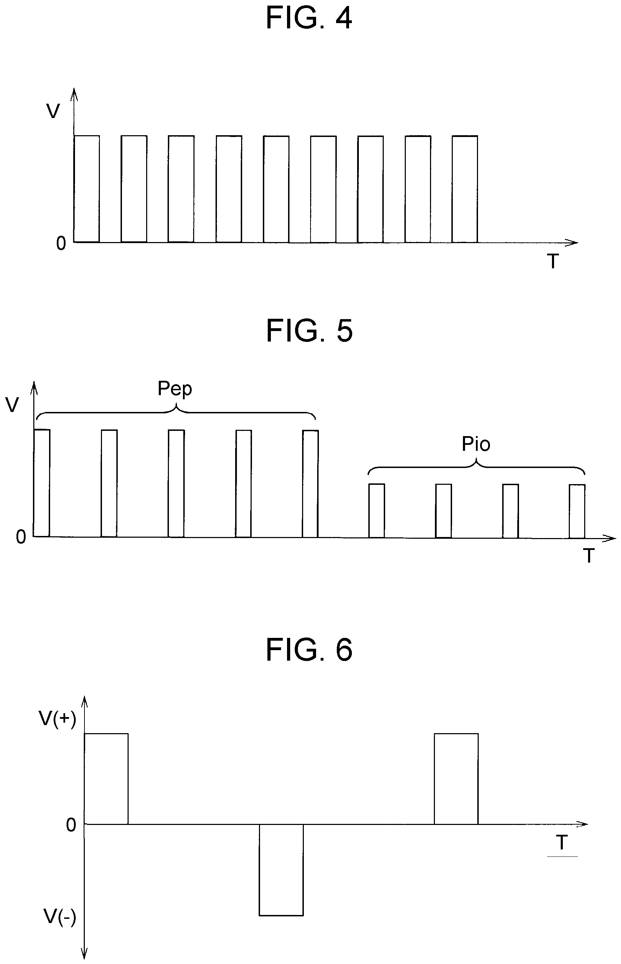

First, Fig. 4 illustrates a waveform chart of a voltage (pulse) which is applied between first skin electrode 21 and grip electrode 33 in the ion introduction mode. In Fig. 4, a vertical axis represents voltage V, and a horizontal axis represents time T.

In the ion introduction mode, controller 36 controls voltage application circuit 35 to energize a DC current between first skin electrode 21 and grip electrode 33.

In the ion introduction mode, a DC current in a direction in which first skin electrode 21 is an anode (positive electrode) and grip electrode 33 is a cathode (negative electrode) is applied to first skin electrode 21 and grip electrode 33.

In the ion introduction mode, for example, voltage V is greater than 0 V and less than or equal to 45 V, a frequency of voltage V (pulse) is between 1 kHz and 5 kHz, and a duty ratio of voltage V (pulse) is between 10% and 90%.

In the ion introduction mode, the user selects the ion introduction mode by using changeover switch 32. The user applies a lotion containing a moisture keeping component and a skin care agent such as a pre-shave lotion to a shaving part (for example, the face of the user), and then shaves hair by using electric razor 10.

In the ion introduction mode, voltage V for ion introduction is applied between first skin electrode 21 and grip electrode 33. As a result, the current flows from first skin electrode 21 to the shaving part, from the shaving part to an arm of the user carrying electric razor 10 through the stratum corneum, and from the arm to grip electrode 33. Thus, the moisture keeping component contained in the skin care agent efficiently permeates the shaving part, and thus, the skin quality of the user is improved.

The shaving part is warmed by warming first skin electrode 21 to a predetermined temperature by using first heater 23, and the moisture keeping component contained in the skin care agent efficiently permeates from the shaving part by a temperature effect (warming effect).

Next, Fig. 5 illustrates a waveform chart of a voltage (pulses) which is applied between second skin electrode 22 and grip electrode 33 or first skin electrode 21, or between first skin electrode 21 and grip electrode 33 in the electroporation mode. Also in Fig. 5, a vertical axis represents voltage V, and a horizontal axis represents time T.

In the electroporation mode, controller 36 controls voltage application circuit 35 to perform energization of electroporation (electric punch method) between second skin electrode 22 and grip electrode 33 or first skin electrode 21.

On the basis of an output of electroporation pulse Pep (see Fig. 5), a DC current in a direction in which second skin electrode 22 is an anode (positive electrode) and grip electrode 33 is a cathode (negative electrode) is applied to second skin electrode 22 and grip electrode 33.

Alternatively, on the basis of the output of electroporation pulse Pep, a DC current in a direction in which second skin electrode 22 is an anode (positive electrode) and first skin electrode 21 is a cathode (negative electrode) is applied to second skin electrode 22 and first skin electrode 21.

When electroporation pulse Pep is output, for example, voltage V is larger than 0 V and less than or equal to 80 V, the frequency of voltage V (pulse) is between 1 kHz and 5 kHz, and the duty ratio of voltage V (pulse) is between 1% and 90%.

On the basis of an output of ion introduction pulse Pio (see Fig. 5), a DC current in a direction in which first skin electrode 21 is an anode (positive electrode) and grip electrode 33 is a cathode (negative electrode) is applied to first skin electrode 21 and grip electrode 33.

When ion introduction pulse Pio is output, for example, voltage V is larger than 0 V and less than or equal to 45 V, the frequency of voltage V (pulse) is between 1 kHz and 5 kHz, and the duty ratio of voltage V (pulse) is between 10% and 90%.

In the electroporation mode, the user selects the electroporation mode by using changeover switch 32. Similarly to a case of the ion introduction mode, the user applies the skin care agent containing the moisture keeping component to the shaving part, and then shaves the hair by using electric razor 10.

First, a voltage of a relatively high voltage (electroporation pulse Pep) is applied between second skin electrode 22 and grip electrode 33 or first skin electrode 21, and an electroporation current flows through the shaving part therebetween. Thus, a lamellar structure of the stratum corneum in the shaving part between second skin electrode 22 and grip electrode 33 or first skin electrode 21 is loosened, and the moisture keeping component of the polymer contained in the skin care agent easily permeates the shaving part.

The voltage for ion introduction (ion introduction pulse Pio) is immediately applied between first skin electrode 21 and grip electrode 33. As a result, the current flows from first skin electrode 21 to the shaving part, from the shaving part to an arm of the user carrying electric razor 10 through the stratum corneum, and from the arm to grip electrode 33. Thus, the moisture keeping component contained in the skin care agent efficiently permeates the shaving part, and thus, the skin quality of the user is improved.

The shaving part is warmed by warming first skin electrode 21 and second skin electrode 22 to a predetermined temperature by using heaters 23 and 24, respectively, and the moisture keeping component contained in the skin care agent efficiently permeates from the shaving part by the temperature effect (heating effect).

Next, Fig. 6 illustrates a waveform chart of a voltage (pulses) which is applied between first skin electrode 21 and grip electrode 33 in the micro-current mode. In Fig. 6, a vertical axis represents voltage V, and a horizontal axis represents time T.

In the microcurrent mode, controller 36 controls voltage application circuit 35 to energize microcurrent (weak current) between first skin electrode 21 and grip electrode 33.

In the microcurrent mode, an AC current is applied between first skin electrode 21 and grip electrode 33.

In the microcurrent mode, for example, voltage V is greater than 0 V and less than or equal to 45 V, the frequency of voltage V (pulse) is between 1 kHz and 5 kHz, and the duty ratio of voltage V (pulse) is between 10% and 90%.

For example, when the skin care agent is not used, the user can select the microcurrent mode. In the microcurrent mode, the user selects the microcurrent mode by using changeover switch 32, and shaves hair by using electric razor 10.

In the microcurrent mode, a voltage of a relatively low voltage is applied between first skin electrode 21 and grip electrode 33. As a result, the current alternately flows from first skin electrode 21 to the shaving part, from the shaving part to the arm of the user having electric razor 10 through the stratum corneum, and from the arm to grip electrode 33. Thus, the skin quality of the user is improved by activating the skin surface of the shaving part.

### [3. Effects and the like]

(1) In the present exemplary embodiment, electric razor 10 includes head 11, grip 12, and blade unit 14 that is disposed in head 11 for cutting hair. Electric razor 10 includes skin electrode (first skin electrode) 21 disposed at a position of head 11 touching a skin surface of a human body during cutting of hair, and grip electrode 33 disposed at a position of grip 12 touching a finger of the human body during cutting of hair. Electric razor 10 further includes voltage application circuit 35 for applying the voltage between first skin electrode 21 and grip electrode 33 to cause the current to flow between first skin electrode 21 and grip electrode 33 through the human body, and controller 36 that controls voltage application circuit 35.

With electric razor 10 having such a configuration, the user can perform shaving and, and energization can be simultaneously performed from the shaving part shaved by the user to the arm holding electric razor 10. Thus, an active component (moisture keeping component) contained in the skin care agent can be more effectively permeated by the shaving part.

(2) As in the present exemplary embodiment, electric razor 10 may further include skin electrode (second skin electrode) 22 disposed in head 11 separately from first skin electrode 21. Controller 36 may have the electroporation mode in which the energization of electroporation is performed to perform the electric punch method between second skin electrode 22 and grip electrode 33 or first skin electrode 21.

As a result, the moisture keeping component of the polymer contained in the skin care agent can permeate into the shaving part, and there is no limitation on the type of the skin care agent which is used during shaving.

(3) An electrical insulation treatment may be performed on a touch portion of second skin electrode 22 with the skin surface of the human body.

As a result, the energization of electroporation can be effectively performed between second skin electrode 22 and grip electrode 33 or first skin electrode 21.

(4) Controller 36 may have the micro-current mode in which a micro-current which is a weak current is energized between first skin electrode 21 and grip electrode 33.

As a result, during normal shaving without using the skin care agent, it is possible to perform the micro-current mode in which the skin surface in the shaving part is cared by causing the current to flow through the shaving part. Thus, even when the skin care agent is not used during shaving, the skin quality of the user can be similarly improved.

(5) Controller 36 may be configured to enable the user to switch between the plurality of modes related to an aspect of the voltage which is applied by voltage application circuit 35.

As a result, the user can appropriately select and switch a mode suitable for the user (skin care mode).

(6) First skin electrode 21 may be formed in a roller shape (see Fig. 9).

As a result, it is possible to prevent a burden from being applied to the shaving part when first skin electrode 21 touches the shaving part.

(7) First skin electrode 21 may have suspension mechanism 25 interposed between first skin electrode 21 and head 11 (see Fig. 7).

As a result, first skin electrode 21 can stably touch the shaving part, and the burden can be prevented from being applied to the shaving part.

(8) First skin electrode 21 may have pop-up mechanism 26 interposed between first skin electrode 21 and head 11 (see Fig. 8).

As a result, since electric razor 10 can be used for skin care other than during shaving, the burden on the skin surface of the user can be reduced during skin care, and skin care for the skin surface other than the shaving part can be performed.

(9) In the present exemplary embodiment, electric razor 10 includes head 11, grip 12, and blade unit 14 that is disposed in head 11 and cuts hair. Electric razor 10 includes skin electrode (first skin electrode) 21 disposed at a position of head 11 touching a skin surface of a human body during cutting of hair, and grip electrode 33 disposed at a position of grip 12 touching a finger of the human body during cutting of hair. Electric razor 10 further includes voltage application circuit 35 for applying the voltage between first skin electrode 21 and grip electrode 33 to cause the current to flow between first skin electrode 21 and grip electrode 33 through the human body, and controller 36 that controls voltage application circuit 35. Heater (first heater) 23 is incorporated in first skin electrode 21.

With electric razor 10 having such a configuration, the user can perform shaving and, and energization can be simultaneously performed from the shaving part shaved by the user to the arm holding electric razor 10. Thus, an active component (moisture keeping component) contained in the skin care agent can be more effectively permeated by the shaving part.

Since first heater 23 is incorporated in first skin electrode 21, the shaving part can be warmed to an appropriate temperature, and the active component contained in the skin care agent can be more effectively permeated by the shaving part. Even in a short time in which hair is shaved with electric razor 10, the active component contained in the skin care agent can be effectively permeated by the shaving part.

Controller 36 includes, for example, a microcontroller having one or more processors and one or more memories. The microcontroller achieves a function as controller 36 by executing a program recorded in one or more memories by one or more processors. The program may be recorded in a memory in advance, may be provided by being recorded in a non-transitory recording medium such as a memory card, or may be provided through an electric communication line. In other words, the program is a program for causing one or more processors to function as controller 36.

Note that, the above exemplary embodiment is to exemplify the techniques in the present disclosure, and therefore, various modifications, replacements, additions, omissions, and the like can be made in the scope of the appended claims or in an equivalent scope thereof.

The present disclosure is applicable to an electric razor that can more effectively reduce a razor loss and more efficiently care for a skin surface for which hair is removed.

### REFERENCE MARKS IN THE DRAWINGS

10, 10A, 10B: electric razor
11: head
12: grip
13: electric razor body
14: blade unit
15: outer net blade (first outer net blade)
16: outer slit blade
17: outer net blade (second outer net blade)
21: skin electrode (first skin electrode)
22: skin electrode (second skin electrode)
23: heater (first heater)
24: heater (second heater)
25: suspension mechanism
26: pop-up mechanism
31: power switch
32: changeover switch
33: grip electrode
34: driver (motor)
35: voltage application circuit
36: controller (controller)

## Claims

1. An electric razor, comprising:
a head;
a grip;
a blade unit that is disposed in the head for cutting hair;
a first skin electrode that is disposed at a position of the head touching a skin surface of a human body during cutting of hair;
a grip electrode that is disposed at a position of the grip touching a finger of the human body during the cutting of hair;
a voltage application circuit for applying a voltage between the first skin electrode and the grip electrode to cause a current to flow between the first skin electrode and the grip electrode through the human body; and
a controller that controls the voltage application circuit.

2. The electric razor according to Claim 1, further comprising:
a second skin electrode that is disposed in the head separately from the first skin electrode,
wherein
the controller has an electroporation mode in which energization of electroporation is performed to perform an electric punch method between the second skin electrode and the grip electrode or the first skin electrode.

3. The electric razor according to Claim 2, wherein
an electrical insulation treatment has been performed on a touch portion of the second skin electrode with the skin surface of the human body.

4. The electric razor according to any one of Claims 1 to 3, wherein
the controller has a micro-current mode in which a micro-current that is a weak current is energized between the first skin electrode and the grip electrode.

5. The electric razor according to any one of Claims 1 to 4, wherein
the controller is configured to enable a user to switch between a plurality of modes relevant to an aspect of the voltage which is applied by the voltage application circuit.

6. The electric razor according to any one of Claims 1 to 5, wherein
the first skin electrode is formed in a roller shape.

7. The electric razor according to any one of Claims 1 to 6, wherein
the first skin electrode has a suspension mechanism interposed between the first skin electrode and the head.

8. The electric razor according to any one of Claims 1 to 7, wherein
the first skin electrode has a pop-up mechanism interposed between the first skin electrode and the head.

9. An electric razor, comprising:
a head;
a grip;
a blade unit that is disposed in the head for cutting hair;
a first skin electrode that is disposed at a position of the head touching a skin surface of a human body during cutting of hair;
a grip electrode that is disposed at a position of the grip touching a finger of the human body during the cutting of hair;
a voltage application circuit for applying a voltage between the first skin electrode and the grip electrode to cause a current to flow between the first skin electrode and the grip electrode through the human body; and
a controller that controls the voltage application circuit,
wherein
a heater is incorporated in the first skin electrode.

10. The electric razor according to Claim 9, further comprising:
a second skin electrode that is disposed in the head separately from the first skin electrode,
wherein the controller has an electroporation mode in which energization of electroporation is performed to perform an electric punch method between the second skin electrode and the grip electrode or the first skin electrode.

11. The electric razor according to Claim 10, wherein
an electrical insulation treatment has been performed on a touch portion of the second skin electrode with the skin surface of the human body.

12. The electric razor according to any one of Claims 9 to 11, wherein
the controller has a micro-current mode in which a micro-current that is a weak current is energized between the first skin electrode and the grip electrode.

13. The electric razor according to any one of Claims 9 to 12, wherein
the controller is configured to enable a user to switch between a plurality of modes.

14. The electric razor according to any one of Claims 9 to 13, wherein
the first skin electrode is formed in a roller shape.

15. The electric razor according to any one of Claims 9 to 14, wherein
the first skin electrode has a suspension mechanism interposed between the first skin electrode and the head.

16. The electric razor according to any one of Claims 9 to 15, wherein the first skin electrode has a pop-up mechanism interposed between the first skin electrode and the head.
